Europäisches Patentamt

European Patent Office

Office européen des brevets

(19)

(11) Veröffentlichungsnummer: **0 337 924**
**A2**

(12) **EUROPÄISCHE PATENTANMELDUNG**

(21) Anmeldenummer: **89730050.5**

(22) Anmeldetag: **28.02.89**

(51) Int. Cl.⁴: **A 61 M 5/14**

(30) Priorität: **13.04.88 DE 3812584**

(43) Veröffentlichungstag der Anmeldung:
**18.10.89 Patentblatt 89/42**

(84) Benannte Vertragsstaaten:
**AT BE CH DE FR GB IT LI LU NL SE**

(71) Anmelder: **MIC MEDICAL INSTRUMENT CORPORATION**
**Friedhofplatz 16**
**CH-4502 Solothurn (CH)**

(72) Erfinder: **Merki, Hans, Dr.med.**
**Rabbentaltreppe 2**
**CH-3013 Bern (CH)**

**Dries, Rolf-Rainer, Dr.**
**Friedhofplatz 16**
**CH-4502 Solothurn (CH)**

(74) Vertreter: **Pfenning, Meinig & Partner**
**Kurfürstendamm 170**
**D-1000 Berlin 15 (DE)**

(54) **Vorrichtung zur Biofeedbackkontrolle von Körperfunktionen.**

(57) Es wird eine Vorrichtung zur Biofeedbackkontrolle von Körperfunktionen mit mindestens einem Sensor zur Erfassung von biologischen Körpergrößen, einem Datenspeicher zur Speicherung von Vergleichswerten, eine Steuereinheit (9,16) und einer Therapieeinrichtung, vorgeschlagen, wobei die Steuereinheit (9,16) die Therapieeinrichtung abhängig von den vom Sensor erfaßten Meßwerten und den Vergleichswerten ansteuert. Die Therapieeinrichtung weist eine Pumpe zur intravenösen, intraarteriellen, oralen oder intraluminalen Applikation und ein mit der Pumpe verbundenes Medikamentenreservoir auf. Die Steuereinheit und/oder die Therapieeinrichtung ist mit einem Speicher versehen, in dem Therapieziele für die Körperfunktionen mit zugeordneten Medikamentendosierungen abhängig von den erfaßten Meßwerten und Vergleichswerten gespeichert sind. Die Pumpe wird derart angesteuert, daß sie aus dem Medikamentenreservoir entsprechend den vorgegebenen Dosierungen das Medikament zu der gewünschten Applikationsstelle fördert, wobei der Sensor die gegebenenfalls auftretende Wirkung des Medikamentes kontinuierlich erfaßt und gemäß der Wirkung eine Veränderung der Ansteuerung der Pumpe vorgenommen wird.

EP 0 337 924 A2

Bundesdruckerei Berlin

# Beschreibung

## Vorrichtung zur Biofeedbackkontrolle von Körperfunktionen

Die Erfindung betrifft eine Vorrichtung zur Biofeedbackkontrolle von Körperfunktionen nach dem Oberbegriff des Hauptanspruchs.

Aus der DE-OS 36 04 986 ist eine Einrichtung zur Verhinderung von Sauerstoffmangelschäden und zur Behebung von Atmungsstörungen bekannt, die mit der Atmung bzw. mit der Sauerstoffversorgung des Gewebes im Zusammenhang stehende Sensoren aufweist. Falls die mit den Sensoren erfaßten Meßwerte vorgegebene Schwellenwerte über-bzw. unterschreiten, werden Schalteinrichtungen angesteuert, die Atemreize durch Anblasen des Patienten mit einer speziellen Atemgasmischung oder Atemweckreize abgeben.

Der Erfindung liegt die Aufgabe zugrunde, eine Vorrichtung gemäß dem Oberbegriff des Hauptanspruchs zu schaffen, die eine kontinuierliche Überwachung von biologischen Körperfunktionen und Therapierung eines Patienten bei automatischer Medikamentenzufuhr gestattet.

Diese Aufgabe wird erfindungsgemäß durch die kennzeichnenden Merkmale des Hauptanspruchs gelöst.

Durch Vorsehen einer Therapieeinrichtung mit mindestens einer Pumpe zur intravenösen, intraarteriellen, oralen oder intraluminalen Applikation und eines mit der Pumpe verbundenes Medikamentenreservoirs, wobei die Pumpe abhängig von vorgegebenen Therapiezielen für die Körperfunktionen und vorgegebenen Medikamentendosierungen abhängig von den gemessenen Werten angesteuert wird und das Medikament zu der gewünschten Applikationsstelle fördert und wobei der Sensor die gegebenenfalls auftretende Wirkung des Medikaments kontinuierlich erfaßt und gemäß der Wirkung eine Veränderung der Ansteuerung der Pumpe vorgenommen wird, kann eine genaue, kontinuierliche und wirksame Therapie unterschiedlicher biologischer Körperfunktionen durchgeführt werden.

Durch die in den Unteransprüchen angegebenen Maßnahmen sind vorteilhafte Weiterbildungen und Verbesserungen möglich.

Ein Ausführungsbeispiel der Erfindung ist in der Zeichnung dargestellt und wird in der nachfolgenden Beschreibung näher erläutert. Die einzige Figur zeigt ein Blockschaltbild der erfindungsgemäßen Vorrichtung.

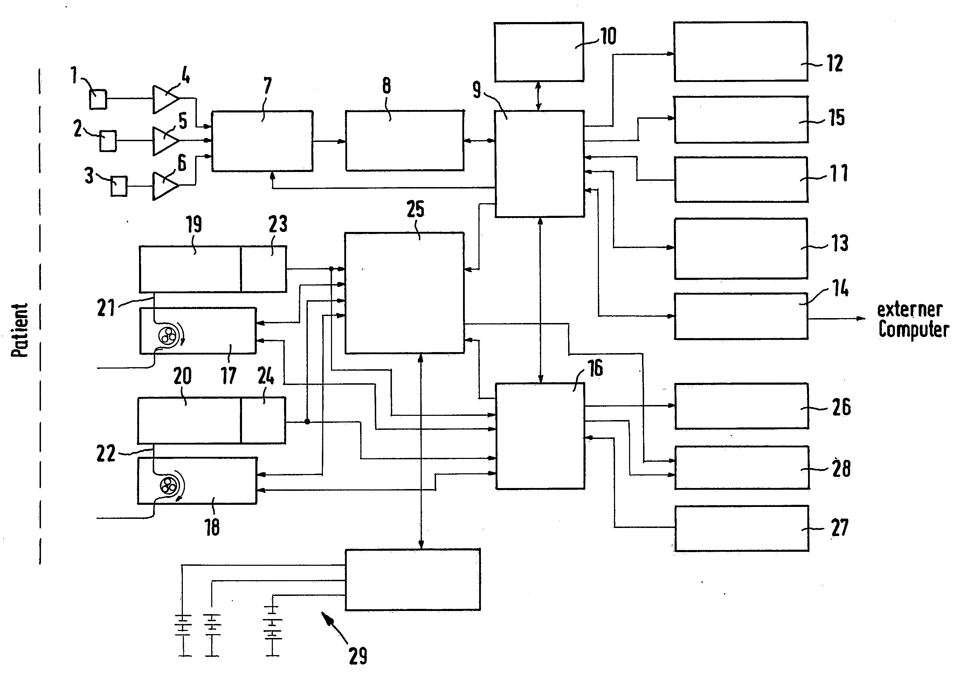

Die in Figur 1 dargestellte Vorrichtung zur Biofeedbackkontrolle weist eine Sensoranordnung auf, die aus einem oder mehreren Sensoren besteht, wobei in der Zeichnung ein pH-Sensor 1, ein Drucksensor 2 und ein Temperatursensor 3 vorgesehen sind. Selbstverständlich können auch andere Sensoren angeschlossen sein, beispielsweise solche, die den Puls, die Atemfrequenz, Potentialdifferenzen oder biochemische Parameter messen. Den Sensoren 1, 2, 3 sind Verstärker 4, 5, 6 zugeordnet, die an einen Multiplexer 7 angeschlossen sind, der über einen A/D-Wandler 8 bzw. direkt mit einem Mikrocomputer 9 verbunden ist. Weiterhin sind ein oder mehrere Datenspeicher 10 vorgesehen, die die von den Sensoren gemessenen Werte speichern und Vergleichs- und Grenzwerte enthalten. Der Mikrocomputer 9 weist ein Tastenfeld 11 auf und ist mit einer alphanumerischen und/oder graphischen Anzeige 12 und einem alphanumerischen und/oder graphischen Drucker 13 verbunden. Weiterhin ist er mit einer Schnittstelle 14 für einen Datentransfer versehen und steuert eine optische und/oder akustische Alarmeinrichtung 15 an.

Neben der in dem oberen Teil der Figur angegebenen Meß- und Auswertevorrichtung 1 bis 15 ist eine Therapieeinrichtung vorgesehen, die mindestens eine von einem weiteren Mikroprozessor 16 angesteuerte Pumpe,in dem dargestellten Ausführungsbeispiel sind es zwei peristaltische Pumpen 17, 18, aufweist, die Medikamente zur intravenösen oder intraarteriellen sowie oralen oder intraluminalen Applikation fördern. Jeder Pumpe 17, 18 ist ein Medikamentenreservoir 19,20 in Form von Einschüben zugeordnet, das die entsprechend der über die Sensoren 1,2,3 und den Mikrocomputer 9 erstellten Diagnose zu applizierende Medikament enthält, wobei an das Medikamentenreservoir 19,20 ein Schlauch 21,22 angeschlossen ist, der über die Pumpe 17,18 zum Patienten geführt wird. Der Einschub jedes Medikamentenreservoirs 19,20 ist mit einer Identifikationskodierung versehen, die von einem Abtaster 23,24, beispielsweise einem optoelektronischen oder magnetischen Abtaster oder dergleichen, erfaßt wird. An den Einschüben der Medikamentenreservoirs 19,20 können auch Füllstandssensoren angeordnet sein, die den jeweiligen Füllstand des Medikamentes in den Einschüben registriert. Anstelle der Füllstandssensoren oder zusätzlich zu ihnen kann der Füllstand auch vom Mikroprozessor 16 festgestellt werden, der die Anfangsmenge des Medikamentes und die jeweils verabreichte Dosierung über die Pumpe 17,18 kennt.

Die Pumpen 17,18 und die Abtaster 23,24 sind sowohl mit dem Mikrocomputer 16 als auch mit einer Überwachungseinheit 25 verbunden, die die Informationen von den Mikrocomputern 9,16 erhält und die erforderliche Redundanz sicherstellt. Die Überwachungseinheit weist vorzugsweise einen eigenen Mikroprozessor auf, wobei für lebensentscheidene Parameter, wie der intraarterielle Druck, eine zweite Sensoranordnung mit Meß-Auswertungseinheit und Vergleichseinheit, die vom Mikroprozessor realisiert werden, vorgesehen sind. Dabei werden die von der Hauptmeß- und -auswerteanordnung 1-9 gemessenen Werte kontinuierlich überprüft. Bei auftretenden Unterschieden, die ein Versagen eines der beiden Systeme wahrscheinlich machen, wird ein optischer und akustischer Alarmimpuls gelöst. Bei nicht lebenswichtigen Parametern, wie Temperatur oder pH-Wert, ist in dem eigenen Mikroprozessor ein Plausibilitätsprogramm enthalten, das die Überwachung des Hauptprogramms übernimmt. Auch für die Therapieeinrichtung sind geeignete Peripheriegeräte, wie Anzeigeeinheit 26, Tastenfeld 27 und optische und/oder akustische Alarmeinheit 28, vorgesehen, die mit dem Mikroprozessor 16 und mit der Überwachungseinheit 25 verbunden sind.

Selbstverständlich können die Mikrocomputer 9, 16 zu einer einzigen Einheit zusammengefaßt sein, wobei dann auch nur ein Tastenfeld, eine Anzeigeeinheit und eine Alarmeinrichtung notwendig sind.

Zusätzlich zur Spannungsversorgung über Netz weist die erfindungsgemäße Vorrichtung eine Spannungsversorgung 29 mit Batterien oder Akkumulatoren auf, die bei Netzausfall den Betrieb auch für längere Zeit (6 bis 8 Stunden) aufrechterhält.

Die Funktion der erfindungsgemäßen Vorrichtung ist folgende:

Die mit den Sensoren 1, 2, 3 über definierte Zeitbereiche gemessenen Werte der biologischen Körpergrößen des Patienten werden in dem Speicher 10 oder in einem anderen Speicher des Mikrocomputers 9 gespeichert und gegebenenfalls rechnerisch beispielsweise zur Bestimmung von Mittelwerten, Medianwerten oder dergleichen umgewandelt. Die Meßwerte werden mit fest vorgegebenen Vergleichswerten bzw. mit von dem Arzt über das Tastenfeld 11 eingegebenen Vergleichswerten verglichen und entsprechend einem im Mikrocomputer 9 gespeicherten Diagnoseprogramm interpretiert.

Im Mikrocomputer 9 bzw. 16 sind Therapieziele, medikamentenhöchstdosen, Dosierungen über bestimmte Zeitbereiche und Art der Medikamentenverabreichung gespeichert. Abhängig von der mit Hilfe der Meßwerte ermittelten Diagnose wird die Therapieeinrichtung aktiviert, d.h. der Mikrocomputer 16 steuert eine oder mehrere Pumpen 17, 18 an, wobei entweder stimulierende oder hemmende Medikamente appliziert werden können. Vor Ansteuerung wird mit Hilfe der die Identifikationskodierung erfassenden Abtaster 23, 24 das jeweilige Medikament identifiziert, so daß die irrtümliche Infusion falscher Medikamente nicht möglich ist. Der Mikrocomputer 16 kann die Pumpen 17, 18 in der Weise ansteuern, daß die Medikamente kontinuierlich, pulsativ oder bolusweise verabreicht werden, wobei auch eine Kombination von pulsatiler Gabe oder kontinuierlicher Gabe mit Bolusgaben möglich ist.

Parallel zu der Medikamentenverabreichung werden weiterhin die interessierenden biologischen Körpergrößen über die Sensoren 1, 2, 3 gemessen, so daß der Erfolg der Therapie kontinuierlich überwacht und gegebenenfalls die Medikamentendarreichung verändert bzw. beendet oder eine anderes Medikament gegeben werden kann.

Je nach Verwendung der Vorrichtung werden die Dosierungsstufen, zum Beispiel vordefinierte Stufen oder vom Mikrocomputer errechnete Dosierungen innerhalb der Tageshöchstmengen sowie der Höchstmengen pro Kurzzeiteinheit, gerätespezifisch für den allgemeinen Anwender fest programmiert oder sind beispielsweise für die Forschung für alle Körperfunktionen frei programmierbar. Die Anzeigeeinheiten 12, 26 ermöglichen die Darstellung von sämtlichen Daten und Funktionen und geben über die gesamte Behandlung von Daten und Therapiefunktionen Auskunft. Entsprechend können über den Drucker 13 alle Daten ausgedruckt werden.

Die Alarmeinrichtungen 15, 28 geben optische und/oder akustische Alarmsignale ab, wenn die Funktionen der einzelnen Bauteile der Vorrichtung, die Angaben des Medikamentenreservoirs, die Diagnosewerte oder die Therapieziele nicht mit den vorgegebenen oder gewünschten Werten übereinstimmen.

Im folgenden sollen zwei Anwendungsbeispiele der erfindungsgemäßen Vorrichtung angegeben werden, und zwar soll erstens eine Magen-pH-Kontrolle über synergistische Therapiefunktionen bei Gleichschaltung der Pumpenfunktionen der Pumpe 17, 18 und zweitens eine Blutdruckkontrolle über antagonistische Therapiefunktionen bei Gegenschaltung der Pumpenfunktionen durchgeführt werden.

1. Magen-ph-Kontrolle: Der pH-Sensor 1 mißt intraluminal die $H^+$-Ionenaktivität, die gemessenen Daten werden abgespeichert und mit dem im Mikroprozessor gespeicherten Werten verglichen. Zur Einstellung des Therapiezieles, beispielsweise Anhebung des pH-Wertes, aktiviert der Mikroprozessor 16 die erste Pumpe mit einem Standardmedikament, zum Beispiel H2-antagonist oder ATPase Hemmer, entweder als "primed infusion", d.h. ein Bolus gefolgt von einer kontinuierlichen Infusion, oder als pulsatile Infusion. Die von der Pumpe applizierte Standarddoses wird schrittweise entsprechend dem Vergleich Therapieziel/Erfolg gesteigert oder reduziert. Die zeitliche Anpassung und Definition des Therapieerfolges/-mißerfolges ist vorgebbar. Bei Verfehlen des Therapiezieles trotz Höchstdosierung wird entweder ein Alarm ausgelöst oder die zweite Pumpe mit einem Kombinationspräparat aktiviert. Die Therapieschritte bei der zweiten Pumpe werden entsprechend denen bei der ersten Pumpe vorgenommen.

2. Blutdruck: Zwei oder drei unabhängige Drucksensoren messen intraarteriell den Druck, der mit dem Zieldruck über definierte Zeitperioden verglichen wird. Die Anpassung des aktuellen Drucks an den Zieldruck erfolgt mit extrem gut steuerbaren Medikamenten, wobei die der ersten Pumpe zugeordneten Medikamente den Druck erhöhen und die der zweiten Pumpe zugeordneten Medikamente den Druck erniedrigen können. Das System kann mit ein bis zwei zusätzlichen, nachgeschalteten Pumpen versehen werden, die zusätzliche synergistische oder antagonistische Medikamente applizieren können. Die Sensorfunktionen werden ständig vom Gerät überwacht und die Meßdaten der zwei bis drei Sensoren werden miteinander verglichen. Bei Auftreten von Abweichungen, die größer als ein vorgegebener Wert sind, wird ein Alarm ausgelöst und die Ansteuerung der Pumpen wird gestoppt.

## Patentansprüche

1. Vorrichtung zur Biofeedbackkontrolle von Körperfunktionen mit mindestens einem Sensor zur Erfassung von biologischen Körpergrößen, einem Datenspeicher zur Speicherung von Vergleichswerten, eine Steuereinheit und einer Therapieeinrichtung, wobei die Steuereinheit die Therapieeinrichtung abhängig von den vom Sensor erfaßten Meßwerten und den Vergleichswerten ansteuert, **dadurch gekennzeichnet**, daß die Therapieeinrichtung eine Pumpe (17,18) zur intravenösen, intraarteriellen, oralen oder intraluminalen Applikation und ein mit der Pumpe (17,18) verbundenes Medikamentenreservoir (19,20) aufweist und daß die Steuereinheit (9,16) und/oder die Therapieeinrichtung mit einem Speicher (16) versehen ist, in dem Therapieziele für die Körperfunktionen mit zugeordneten Medikamentendosierungen abhängig von den erfaßten Meßwerten und Vergleichswerten gespeichert sind und daß die Pumpe (17,18) derart angesteuert wird, daß sie aus dem Medikamentenreservoir (19,20) entsprechend den vorgegebenen Dosierungen das Medikament zu der gewünschten Applikationsstelle fördert, wobei der Sensor (1,2,3) die gegebenenfalls auftretende Wirkung des Medikamentes kontinuierlich erfaßt und gemäß der Wirkung eine Veränderung der Ansteuerung der Pumpe (17,18) vorgenommen wird.

2. Vorrichtung nach Anspruch 1, dadurch gekennzeichnet, daß die Pumpe (17,18) els peristaltische Pumpe ausgebildet ist.

3. Vorrichtung nach Anspruch 1 oder 2, dadurch gekennzeichnet, daß mehrere Pumpen mit zugeordneten Medikamentenreservoirs (19,20) vorgesehen sind, wobei die in den Medikamentenreservoirs aufgenommenen Medikamente eine synergistische oder eine antagonistische Wirkung haben.

4. Vorrichtung nach einem der Ansprüche 1 bis 3, dadurch gekennzeichnet, daß der Sensor als pH-Sensor (1), Drucksensor (2), Temperatursensor (3) oder dergleichen ausgebildet ist.

5. Vorrichtung nach einem der Ansprüche 1 bis 4, dadurch gekennzeichnet, daß mehrere Sensoren zur Messung unterschiedlicher biologischer Körpergrößen vorgesehen sind.

6. Vorrichtung nach einem der Ansprüche 1 bis 5, dadurch gekennzeichnet, daß mehrere Sensoren gleichen Typs vorgesehen sind, deren Meßdaten miteinander verglichen werden und bei Auftreten von Abweichungen über einen vorgegebenen Wert eine Alarmeinrichtung (15,28) ausgelöst wird.

7. Vorrichtung nach einem der Ansprüche 1 bis 6, dadurch gekennzeichnet, daß die mindestens eine Pumpe (17,18) kontinuierlich, pulsatil oder bolusweise aktiviert wird.

8. Vorrichtung nach Anspruch 1, dadurch gekennzeichnet, daß das Medikamentenreservoir (19,20) mit einer Identifizierungsmarkierung versehen ist, die von einem Abtaster (23,24) abgetastet wird.

9. Vorrichtung nach Anspruch 1 oder 8, dadurch gekennzeichnet, daß der Füllstand des Medikamentenreservoirs erfaßt wird.

10. Vorrichtung nach einem der Ansprüche 1 bis 19, dadurch gekennzeichnet, daß zur Sicherstellung der erforderlichen Redundanz eine Überwachungseinheit (25) vorgesehen ist.

11. Vorrichtung nach Anspruch 10, dadurch gekennzeichnet, daß für lebensentscheidende Körpergrößen, z.B. den intraarteriellen Druck, die Überwachungseinheit (25) eine unabhängige zweite Sensoranordnung mit Auswerteeinheit und eine Vergleichsvorrichtung aufweist, durch die die Hauptmeß- und -auswerteanordnung (1-9) überwacht wird.

12. Vorrichtung nach den Ansprüchen 10 oder 11, dadurch gekennzeichnet, daß für nicht lebenswichtige Körpergrößen, wie pH-Wert oder Temperatur, die Überwachungseinheit (25) einen zugeordneten Mikroprozessor mit einem Plausibilitätsprogramm aufweist.